(19) Europäisches Patentamt
European Patent Office
Office européen des brevets

(11) Publication number: 0 318 948 B1

(12) # EUROPEAN PATENT SPECIFICATION

(45) Date of publication of patent specification: **19.08.92**

(51) Int. Cl.[5]: **A61K 47/00**, A61K 39/395, A61K 49/02

(21) Application number: **88119966.5**

(22) Date of filing: **30.11.88**

(54) **Cleavable immunoconjugates for the delivery and release of agents in native form.**

(30) Priority: **02.12.87 US 127656**

(43) Date of publication of application:
**07.06.89 Bulletin 89/23**

(45) Publication of the grant of the patent:
**19.08.92 Bulletin 92/34**

(84) Designated Contracting States:
**DE FR GB IT SE**

(56) References cited:
**EP-A- 0 115 171**
**WO-A-86/01409**

**Phytochemistry 1979, vol. 18, pp. 666-667, Banthorpe et al.**

(73) Proprietor: **NEORX CORPORATION**
**410 West Harrison Street**
**Seattle Washington 98119(US)**

(72) Inventor: **Hylarides, Mark D.**
**5309 - 104th S.W.**
**Everett Washington 98204(US)**
Inventor: **Srinivasan, Ananthachari**
**11420 - 109th Avenue N.E.**
**Kirkland Washington 98033(US)**
Inventor: **Fitzner, Jeffrey N.**
**10740 Greenwood Avenue North No. 5**
**Seattle Washington 98133(US)**

(74) Representative: **Brown, John David et al**
**FORRESTER & BOEHMERT Franz-Joseph-Strasse 38**
**W-8000 München 40(DE)**

EP 0 318 948 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates generally to cleavable immunoconjugates that permit release of an agent in native form under mild conditions and to methods for making and using these conjugates.

A reoccurring problem in medicine is that, due to the lack of specificity of the agents used for treatment of illnesses, the patient is often the recipient of a new set of maladies from the therapy. This scenario is common especially in the treatment of the various forms of cancer.

An approach taken to circumvent the nonspecificity of the agents used to treat diseases is to couple an agent to a carrier that possesses some degree of specificity. A number of molecules have been utilized as carriers in agent delivery systems, but with limited success. Carrier molecules such as liposomes, proteins, and polyclonal antibodies have been used in conjunction with a broad spectrum of pharmaceutical or cytotoxic agents including radioactive compounds, agents which bind DNA, antimetabolites, agents which act on cell surfaces, and protein synthesis inhibitors.

With the discovery of a method to isolate antibodies with a single specificity, i.e., monoclonal antibodies (MAbs), came the hope that agents could now be delivered to selected cells via "immunoconjugates." "Immunoconjugates" are covalently bonded hybrid molecules composed of a recognition portion, such as an antibody molecule, an antibody fragment, or a functional equivalent thereof, and a biologically active portion, such as a toxin, toxin fragment, a drug, a biological response modifier, or a radioisotope. Immunoconjugates have enormous potential as potent anti-tumor agents, due to the selectivity imparted to the hybrid molecules by the antibody portion of the immunoconjugate. The exquisite selectivity of antibodies or antibody fragments permits delivery of increased doses of cytotoxic, inhibitory or radiolabeled moieties to a defined population of cells.

Although the MAb carrier systems have gone far to solve the cell-specificity problem, other problems remain. In particular, the design of the compound used to link the agent to the MAb is important. First, where the agent is only active, or at least more potent, when free from the MAb carrier, the linker needs to be cleavable in order to release the agent. Second, where the agent is only active, or at least more potent, when none of the linker remains attached following the cleavage, the labile bond must be the one formed between the linker and the agent. Third, the type of labile bond used should be chosen on the basis of the location, i.e., inside or outside a cell, of the release-inducing factor.

A number of different cleavable linker groups have been described previously. The mechanisms for release of an agent from these linker groups include cleavage by reduction of a disulfide bond, by irradiation of a photolabile bond, by hydrolysis of derivatized amino acid side chains, by serum complement-mediated hydrolysis, and acid-catalyzed hydrolysis. Some of these mechanisms are susceptible to release of the agent prior to having reached the specific cell, tissue or organ. Other of these mechanisms will provide faithful external delivery, however, they are inappropriate where the actual target site of the agent is inside a cell. Where an agent activates or inactivates a cell by binding to an intracellular component, the carrier-agent conjugate must be internalized and then the agent released.

A way to achieve internalization of a carrier-agent conjugate is to take advantage of a cell's receptor-mediated endocytosis pathway. Antibodies are one example of a carrier that will bind to cell surface receptors and be internalized. Receptors which are internalized by receptor-mediated endocytosis pass through acidified compartments known as endosomes or receptosomes. Thus, the carrier-agent conjugate will be exposed transiently to an acidic pH.

Blattler et al., in U.S. Patent No. 4,569,789, describe a drug delivery system which is formed by reaction of an active substance with a maleic anhydride moiety. The active substance is released upon cleavage of the amide bond. The patent purports that cleavage occurs under mildly acidic conditions, yet the patent discloses that at pH 5 only about, 15% is cleaved after five hours. Even at pH 4 for five hours, less than 50% is cleaved.

Thus, there is a need in the art for a carrier-agent conjugate which releases the agent by cleavage under mild conditions. The present invention fulfills this need and further provides other related advantages.

Briefly stated, the present invention, in one aspect, provides for a method of producing cleavable immunoconjugates comprising the steps of reacting an agent with a compound having the formula (I):

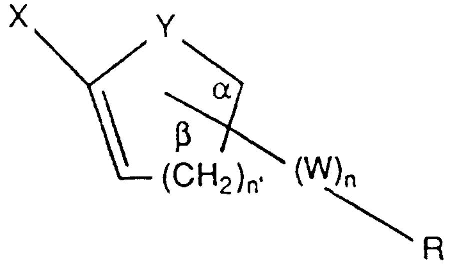

(I)

where:

R is a chemically reactive moiety;

W is a methylene, methylenoxy, or methylenecarbonyl group or combination thereof;

n is 0 to 10;

Y is an O, S, or NR', wherein R' is an alkyl group of $C_6$ or less;

n' is 1 to 2; and

X is an H, alkyl group of $C_6$ or less, or alkoxy group of $C_6$ or less.

The agent adds to the carbon-carbon double bond, thereby forming a derivatized agent. The derivatized agent is conjugated with an antibody or antibody fragment for delivery to a cell, thereby forming the cleavable immunoconjugate. A variation on this method is to reverse the order of addition of an agent to the compound and of conjugation of an antibody.

In another aspect, the invention provides a cleavable immunoconjugate. The immunoconjugate has the formula (II):

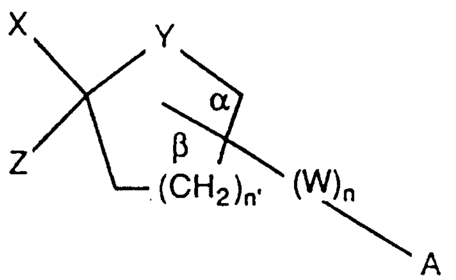

(II)

where:

A is an antibody including the linking function, or antibody fragment including the linking function;

W is a methylene, methylenoxy, or methylenecarbonyl group or combination thereof;

n is 0 to 10;

Y is an O, S or NR', wherein R' is an alkyl group of $C_6$ or less;

n' is 1 to 2;

X is H, alkyl group of $C_6$ or less, or alkoxy group of $C_6$ or less; and

Z is an agent.

In yet another aspect, the present invention provides the use of said immunoconjugates in a method for delivering to the cytoplasm of a target cell an agent free of its antibody carrier. Such a method comprises the step of administering to a mammal a diagnostically or therapeutically effective dose of a cleavable immunoconjugate having the formula (II):

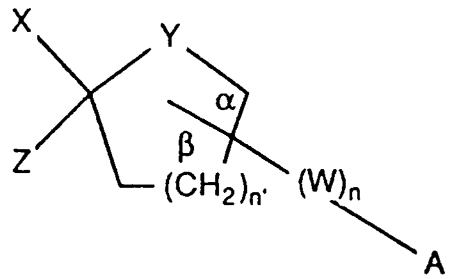

(II)

where:
A is an antibody including the linking function, or an antibody fragment including the linking function;
W is a methylene, methylenoxy, or methylenecarbonyl group or combination thereof;
n is 0 to 10;
Y is an O, S or NR', wherein R' is an alkyl group of $C_6$ or less;
n' is 1 to 2;
X is H, alkyl group of $C_6$ or less, or alkoxy group of $C_6$ or less; and
Z is an agent.
Upon delivery of the immunoconjugate to a target cell, the bond joining the agent to the conjugate is cleaved, thereby releasing the agent. The bond is cleavable under a variety of conditions, including mildly acidic conditions or divalent cations, and is accelerated by heat. Since the agent may be bonded via one of its nucleophilic groups directly to the linker, cleavage can result in release of native agent.

Other aspects of the invention will become evident upon reference to the following detailed description.

As noted above, the present invention provides cleavable immunoconjugates and a method for producing the immunoconjugates. Conjugates in which the carrier is an antibody, fragment thereof, or functional equivalent thereof, have enormous potential as potent anti-tumor agents. This is due to the selectivity imparted to the conjugate by the antibody portion. The exquisite selectivity of antibodies permits delivery of increased doses of agents, such as those that are cytotoxic, inhibitory, radiolabeled, or biological response modifiers.

One advantage of the immunoconjugates described is that cleavage occurs under mildly acidic conditions. For example, subjecting a conjugate to pH 5 results in substantially complete cleavage. The release mechanism may be described using as a model the example where as oxygen is the ring heteroatom. An equivalent of acid is thought to initially protonate the ring O. This is followed by formation of a stable carbocation. A second equivalent of acid results in the release of the agent and the formation of an aldehyde or ketone on the compound linking the agent to the antibody.

Another advantage of the immunoconjugates of the present invention is that cleavage results in the release of the agent without any of the linker remaining attached. Popular cleavable linkers are those bifunctional reagents with a disulfide bond interposed between two reactive end groups. Cleavage of the disulfide bond by the addition of a reducing compound leaves one end of the bifunctional reagent still attached to the agent. Many agents, however, are inactivated by the permanent addition of a linker or fragment thereof to their structures. The immunoconjugates of the present invention are cleaved at the bond formed between the agent and the compound linking the agent to the antibody. Therefore, the present invention provides a way of releasing an agent in native form at the target site.

Yet another advantage of the present invention is the ease of preparation of the linking compound, due in part to the commercial availability of the reagents needed.

The present invention also provides a method for producing a cleavable immunoconjugate comprising the steps of reacting an agent, capable of addition to a carbon-carbon double bond, with a compound having the formula (I):

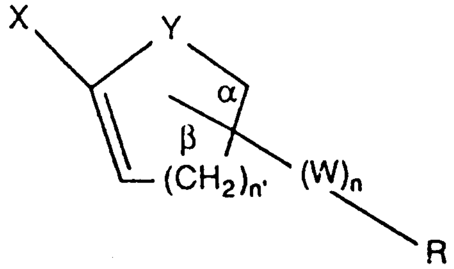

(I)

to form a derivatized agent and conjugating the derivatized agent with an antibody or fragment thereof to form the cleavable immunoconjugate.

The elements of the compound depicted above in formula I include the following. R is a chemically reactive moiety. The moiety may be strongly electrophilic or nucleophilic and thereby be available for reacting directly with an antibody or fragment thereof. Alternatively, the moiety may be a weaker electrophile or nucleophile and therefore require activation prior to the conjugation with an antibody or fragment thereof. This alternative would be desirable where it is necessary to delay activation of R until an agent is added to the compound in order to prevent the reaction of the agent with R. In either scenario R is chemically reactive, the scenarios differ by whether following addition of an agent, R is reacted directly with an antibody or fragment thereof or is reacted first with one or more chemicals to render R capable of reacting with an antibody or fragment thereof. A discussion of reactions illustrative of the activation of R is found below.

W in formula I is a group that functions as a "spacer arm" and may be useful to distance the antibody or fragment thereof from the agent. Groups which may be used include methylene ($-CH_2-$), methyleneoxy ($-CH_2-O-$), methylenecarbonyl ($-CH_2-CO-$), amino acids, or combinations thereof. The number, n, of groups such as these would be typically 0 to 30 and preferably 0 to 10. W, or R where n is 0, may be attached to one or the other of the ring positions designated as $\alpha$ and $\beta$. Because the number of methylene ring carbons at the $\beta$ position is defined by $n'$ which may be greater than one, the $\beta$ position includes additional points for attachment of a W or an R to the ring.

Y in formula I is a heteroatom. Preferred heteroatoms include oxygen (O), sulfur (S), or nitrogen (N). When nitrogen is the heteroatom, it should be in the form of a tertiary amine, i.e., $NR'$, such as where $R'$ is an alkyl group of $C_6$ or less. A particularly preferred heteroatom is O. The ring size of the compound may be increased above 5 by an increase in the number, $n'$, of ring methylene groups. Preferred are 5-membered rings, such as a dihydrofuran derivative, and 6-membered rings. X may be a hydrogen (H) or another substituent, preferably an alkyl group of $C_6$ or less and an alkoxy group of $C_6$ or less.

The step of reacting an agent and a compound of formula I results in the attachment of the agent to the compound via addition to the carbon-carbon double bond on the ring. In this manner, a derivatized agent is formed. Any agent containing a group capable of reacting with the compound may be employed in the present invention. Preferred agents include drugs, toxins, biological response modifiers, radiodiagnostic compounds and radiotherapeutic compounds. An agent may be reacted in its native form or a derivative thereof. An example of a derivative form is where an amino group on an agent is modified by reaction with a compound, such as iminothiolane, to introduce a sulfhydryl group on the agent. Preferred toxins include ricin, abrin, diptheria toxin and Pseudomonas exotoxin A. Preferred radionuclides for the radiodiagnostic and radiotherapeutic compounds include $^{99m}Tc$, $^{186/188}Re$, and $^{123/131}I$.

The step of conjugating may be performed by joining an antibody or fragment thereof to the derivatized agent by direct reaction with R. Alternatively, it may be desirable to include before the step of conjugation a preparatory step. For example, an antibody or fragment thereof may be itself derivatized in preparation for direct reaction with R. The derivatization of an antibody or antibody fragment includes reaction with any of the numerous bifunctional reagents reported in the literature.

A direct reaction with R by derivatized or underivatized antibody or fragment thereof is intended to mean that R is capable of reacting with the derivatized or underivatized antibody or fragment. For example, R may be a carbonyl-containing group, such as an anhydride or an acid halide, or an alkyl group containing a good leaving group, e.g., a halide. The latter class of compounds may be represented by alkyl X', where X' stands for the leaving group. As another example, R may be a nucleophilic group, such as an amino or sulfhydryl group, which is capable of reacting with a derivatized antibody or fragment, e.g., containing a maleimide group.

Yet another way to perform a step in preparation for conjugation of the derivatized agent with

underivatized or derivatized antibody or antibody fragment is to convert R. Examples of conversions of R include where R is a carboxyl group and is then activated. Activation of a carboxyl group includes formation of an "active ester," such as a succinimidyl ester. The term "active ester" is known to refer to esters which are highly reactive in nucleophilic substitution reactions. In the present invention, the antibody or antibody fragment would be the nucleophile.

Another example of a conversion is where R is a succinimide derivative containing a protective group, such as phenylsulfonyl. Upon removal of the group, the succinimide is converted to a maleimide which is highly reactive in nucleophilic addition reactions. Alternatively, R may be an amino, sulfhydryl, or hydroxyl group and the conversion comprises reaction with a bifunctional reagent. It will be evident to one skilled in the art that a variety of bifunctional reagents, both homobifunctional and heterobifunctional, may be employed within the present invention.

The antibody employed in the present invention may be an intact molecule, a fragment thereof, or a functional equivalent thereof. Examples of antibody fragments are $F(ab')_2$ , Fab', Fab, and Fv. While polyclonal antibodies may be employed in the present invention, monoclonal antibodies (MAbs) are preferred, especially those directed toward a tumor-associated antigen in man. Particularly preferred MAbs are anti-TAC, or other interleukin 2 receptor antibodies; 9.2.27 and NR-ML-05 to human melanoma associated proteoglycan; NR-Lu-10 to 37-40 kilodalton pancarcinoma glycoprotein and $OVB_3$ to an as yet unidentified antigen. Genetically engineered antibodies or fragments thereof of these and other MAbs may be employed as well.

A variation on this method is to reverse the order of the addition of an agent and an antibody in the formation of an immunoconjugate. Specifically, first an antibody is conjugated to a compound, whose general structure is depicted above, via R and then an agent is reacted via addition to the double bond on the compound attached to the antibody. The above discussion regarding the compound, agent, antibody, and chemical reactions is relevant to this variation as well.

In addition to the methods provided, the present invention provides cleavable immunoconjugates having the formula (II):

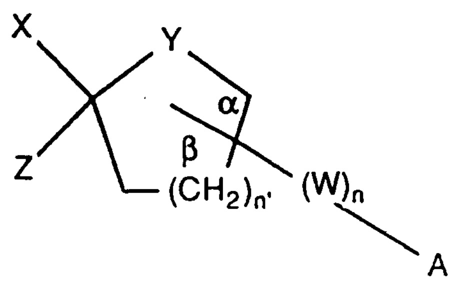

(II)

where: W, n, Y, n', $\alpha$, $\beta$, and X are defined as described above. A is an antibody or an antibody fragment, and for either it includes any linking function used to attach the antibody or fragment to yield the immunoconjugate. Preferred MAbs are described above. Z is an agent. Any agent capable of being covalently attached to the cleavable immunoconjugate may be employed in the present invention. Typically, an agent will be bonded to the immunoconjugate by use of a sulfhydryl, amino, or hydroxyl group on the agent. The agent may be bonded directly to the immunoconjugate or indirectly with a linking function interposed between the agent and the ring. Preferred agents include those described above.

An additional aspect of the present invention is its use in a method for delivering to the cytoplasm of a target cell an agent free of its antibody carrier. Such a method comprises the step of administering to a mammal a diagnostically or therapeutically effective dose of a cleavable immunoconjugate having the formula (II):

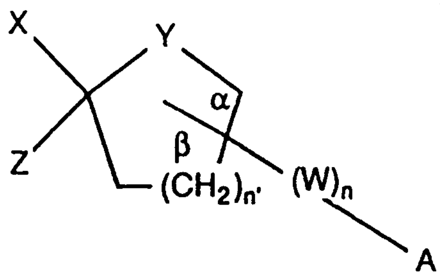

(II)

where W, n, Y, n', $\alpha$, $\beta$, X, A, and Z are defined above. A preferred mammal is man. Preferred MAbs and agents include those described above.

The agent may be diagnostically and/or therapeutically effective. A preferred diagnostic agent is a compound containing $^{99m}Tc$. A diagnostically effective dose of a cleavable immunoconjugate incorporating such an agent is generally from 10 to 30, typically from 15 to 25, and preferably from 0.666 GBq to 0.74 GBq (18 to 20 mCi) per 75 kg body weight. A preferred therapeutic agent is a toxin, such as Pseudomonas exotoxin A. A therapeutically effective dose of a cleavable immunoconjugate incorporating such as agent is generally from 1 to 100 and preferably from 1 to 10 ng per 75 kg body weight. The precise dose for a particular cleavable immunoconjugate is dependent upon the antibody used, as antibodies vary with respect to the number of receptors and their affinity for the receptors, and the agent used, as toxins, for example, vary with respect to their potency. It will be evident to one skilled in the art how to determine the optimal effective dose for a particular cleavable immunoconjugate.

The step of administering to a mammal a diagnostically or therapeutically effective dose of a cleavable immunoconjugate having formula II sets in motion a sequence of events in vivo that results in the agent portion of the immunoconjugate being delivered free of the antibody portion to the cytoplasm of a target cell. The antibody or antibody fragment portion of the immunoconjugate imparts the selectivity which permits delivery to and binding at the surface of a specific cell. An immunoconjugate of formula II is susceptible to cleavage by pH less than or equal to 6.0 and the acid-catalyzed cleavage is accelerated by heating above room temperature, about 23°. Since antibodies bind to cell surface receptors which are internalized into the cytoplasm via acidified compartments, it is believed that the release to the cytoplasm of an agent from an immunoconjugate of the type described herein is the result of this transient exposure to acidic pH. Futher, because mammals such as man have normal body temperatures above 23°C, body heat may be a factor in the release. The delivery of an agent free of its antibody carrier to the cytoplasm of a targeted cell increases its potency as compared to the agent when irreversibly linked to its carrier. This method is useful to diagnose, stage, evaluate or treat diseases such as cancer in humans.

To summarize the examples which follow, Example I provides the preparation of cleavable immunoconjugates utilizing 6-mercaptopurine and MAb 9.2.27. Example II describes testing for the in vitro cytotoxicity and the biodistribution and toxicology of the cleavable immunoconjugates utilizing Pseudomonas exotoxin. Example III discloses isolation of phenylmercaptoacetamide utilizing a derivatized agarose.

## EXAMPLES

### EXAMPLE I

### Preparation of Cleavable Immunoconjugates

A. Preparation of a Derivatized Agent

The derivatized agent having the following formula is prepared as described below:

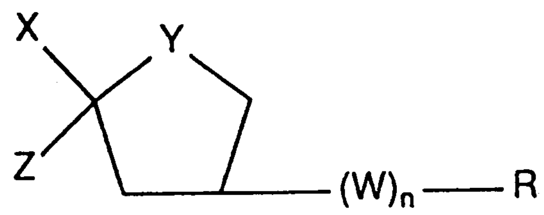

where:

R =

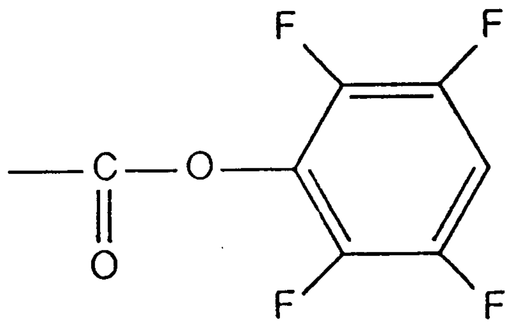

W = $-CH_2-$
n = 1
Y = oxygen
X = $CH_3$
Z = 6-mercaptopurine

1. Preparation of 2,3-dihydro-5-methyl-furan-3-yl acetic acid 1.

Compound 1 where n′ = 1 is synthesized according to the procedure described by D.V. Banthorpe et al. (Phytochemistry 18:666-667, 1979) as depicted in Scheme I.

Scheme I

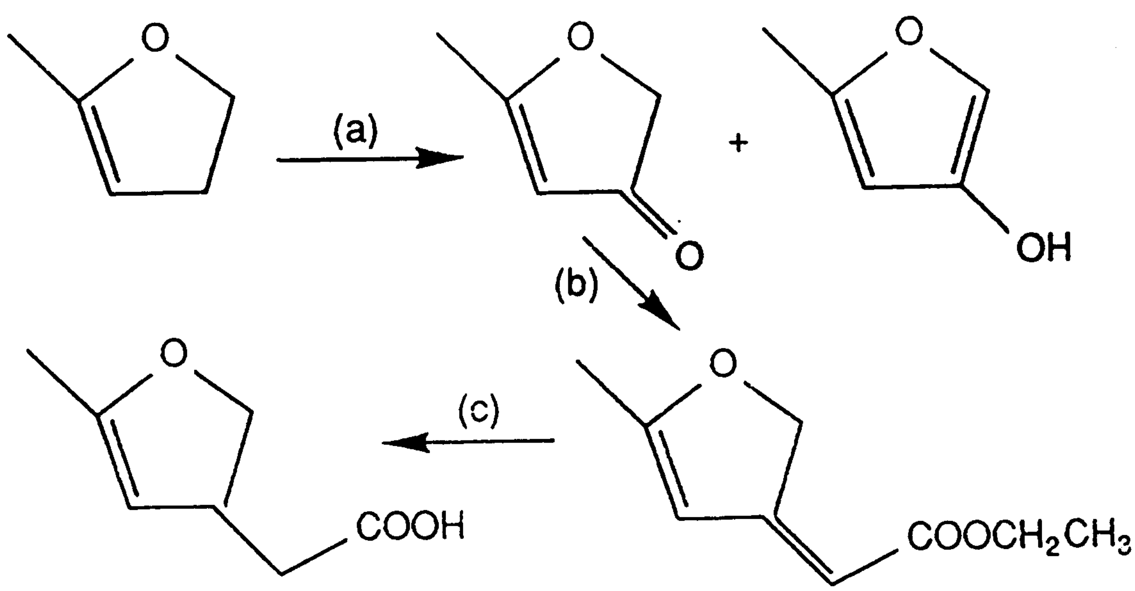

(a) $Se0_2$, dioxane
(b) $CH_3CH_2OOCH=PO_3$
(c) Raney nickel

Similarly, the dihydropyran derivative 1B where n' = 2 is prepared using the analogous method described in Scheme I.

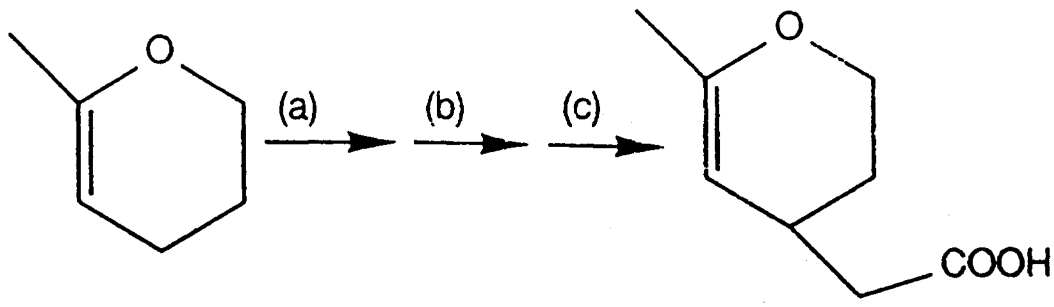


2. Reaction of An Agent with Compound 1.

A hemithioketal derivative, 2, of 6-mercaptopurine, an anti-tumor drug, where n' = 1 is prepared according to the pathway depicted in Scheme II below. Specifically, a mixture of 2,3-dihydro-5-methyl-furan-3-yl acetic acid 1 (1.0 equivalent) and 6-mercaptopurine (1.0 equivalent) in THF is treated with a catalytic amount of para-toluene sulfonic acid (0.01 equivalent). The resultant mixture is allowed to stir at room temperature overnight. The organic phase is washed with $H_2O$ and dried over anhydrous $MgSO_4$. After removal of the solvent, a hemithioketal derivative of 6-mercaptopurine, product 2, is obtained.

Scheme II

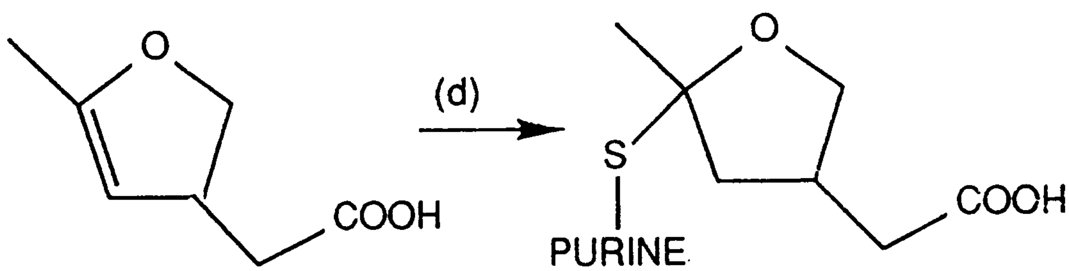


1 2

(d) 6-mercaptopurine, pTsOH, THF

3. Conversion of 2 to the tetrafluorophenyl ester 3.

An activated ester form, 3, of 2 is prepared according to the pathway depicted in Scheme III below. Specifically, the hemithioketal derivative of 6-mercaptopurine, 2, (1.0 equivalent) is combined with 2,3,5,6 tetrafluorophenol (TFP) (1.1 equivalent) in anhydrous THF. After addition of crystalline dicyclohexyl carbodiimide (DCC) (1.1 equivalent), the mixture is stirred for 12 hours at 25 ° C. After removal of the precipitated dicyclohexyl urea by filtration and removal of the THF under reduced pressure, the product is obtained. After chromatography on silica gel, product 3 is obtained.

Scheme III

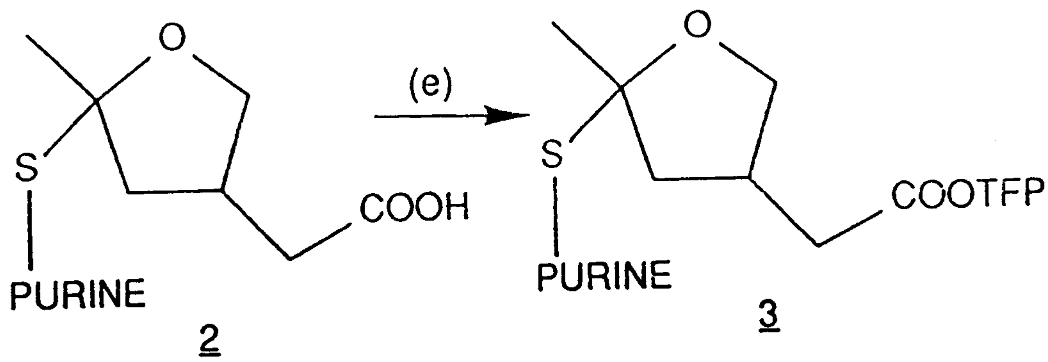


2

3

(e) 2,3,5,6-tetrafluorophenol, DCC, THF

B. Conjugation of TFP ester 3 with Monoclonal Antibody 9.2.27

The monoclonal antibody 9.2.27 is directed to a melanoma-associated antigen and is prepared to according to Morgan et al., Hybridoma 1:27-35, 1981. A methanolic solution of 3 (25 μl total volume) is transferred to a vial containing buffered antibody solution (pH 8.5-10) of at least 1 mg antibody per 1 ml. The conjugation mixture is warmed at 37°C for 20 minutes. The modified antibody is purified either using gel permeation chromatography or small pore filtration (e.g., Centricon ultra centrifugation).

EXAMPLE II

A. In Vitro Cytotoxicity of Cleavable Immunoconjugates

Cleavable immunoconjugates, where Pseudomonas exotoxin (PE) is the agent, are prepared in accordance with a modification of the procedures of Example I.

Prior to reaction with compound 1 (Example I.A.2.), the carboxyl groups on PE are protected, e.g., by suspension of PE in O.1N methanolic HC1 at room temperature overnight. PE is then treated with a reducing agent to provide a free sulfydryl for reaction with compound 1. A mixture of compound 1 (1.0 equivalent) and PE (1.0 equivalent) in an aqueous solution containing 10% dioxane is treated with a catalytic amount of para-toluene sulfonic acid (0.01 equivalent). The resultant mixture is allowed to stir at room temperature overnight and the derivatized PE is isolated by gel filtration chromatography. The succinimidyl ester of derivatized PE is prepared using N-hydroxysuccinimide and a water soluble carbodiimide. After separation by gel filtration chromatography, the carboxyl groups on PE are deblocked, e.g., by hydroxylamine, and PE is conjugated to Mab 9.2.27 according to Example I.B.

ADP-ribosylation is measured in a cell-free system according to the method of B.G. Vanness et al., J.Biol. Chem. 255:10717 (1980). The ADP-ribosylation activity of the cleavable PE immunoconjugates in the presence and absence of dilute acid or divalent cations is compared to the activities under the same conditions of PE alone and of non-cleavable PE immunoconjugates.

In vitro cytotoxicity testing is performed according to the method of A.C. Morgan, Jr. et al., JNCI 78:1101, 1987, using $^{3}$H-leucine incorporation to measure protein synthesis inhibition due to ADP-ribosylation activity by PE. For testing of PE:9.2.27 conjugates, two human melanoma cell lines are utilized as targets - A375 met mix (antigen-positive) and A375 I$^{0}$ Primary (antigen-negative). For assay of PE:anti-TAC conjugates, target cells are HUT 102 (antigen-positive) and CEM (antigen-negative) as discussed in D.J.P. Fitzgerald et al., J. Clin. Invest. 74:966 (1984). Conjugates are examined in two formats: (a) short exposure, wherein the conjugate is incubated with target cells for one hour at 37°C, the monolayer gently washed, and the cultures continued for up to 72 hours before the addition of $^{3}$H-leucine; and (b) long exposure, wherein the conjugate is added and the target cells exposed for the entire length of the culture period.

B. Biodistribution and Toxicology of Cleavable Immunoconjugates

Tumor localization and biodistribution of conjugates are examined in a nude mouse xenograft model of human melanoma, according to the method of K.M. Hwang et al., Canc. Res. 450:4150, 1985. PE is radiolabeled with $^{125}$I-para-iodophenyl (PIP) as shown by D.S. Wilbur et al., J. Nucl. Med. 27:959 (1986). This radiolabel is not subject to dehalogenation, and thereby can be used to more effectively follow the biodistribution of conjugates. The radiolabeled PE is conjugated to a monoclonal antibody, such as 9.2.27 or anti-TAC, using the linkers of the present invention as described in Example I. Animals are sacrificed at 20 hours post-injection, and organs are blotted, weighed and counted. A %dose per gram is calculated for each tissue. In addition, serum half-life is estimated by retroorbital sampling of whole blood.

Mice are administered different doses of PE-anti-TAC and PE:9.2.27 conjugates intraperitoneally to determine an $LD_{100}$. Following administration of radiolabeled PE immunoconjugates, the resultant tumor localization and biodistribution of the conjugates are determined. Non-specific toxicity of PE:anti-TAC conjugates is also assessed in cynomolgous monkeys. Monkeys are monitored for liver enzyme levels, and are observed for other relevant symptoms, including appetite, presence/absence of nausea, and temperature.

EXAMPLE III

Isolation of a Compound Containing an Available Nucleophilic Group By a Derivatized Solid Phase

A. Preparation of a Derivatized Solid Phase

1. Synthesis of Reagent, 4, for Derivatization of Solid Phase.

Compound 1, 2,3-dihydro-5-methyl-furan-3-yl acetic acid, is synthesized as described in Example I. To a solution of 1 (1.0 equivalent) in dry dimethylformamide (DMF) is added crystalline dicyclohexyl carbodiimide (DCC) (1.1 equivalent), followed by the addition of N-hydroxysulfosuccinimidate (1.1 equivalent). After stirring at room temperature for 12 hours, the precipitated dicyclohexylurea (DCU) is removed by gravity filtration. The DMF is removed under reduced pressure and the resultant residue is dissolved in a minimal amount of 50:50 acetonitrite-$H_2O$. This compound 4 is then purified using silica gel chromatography.

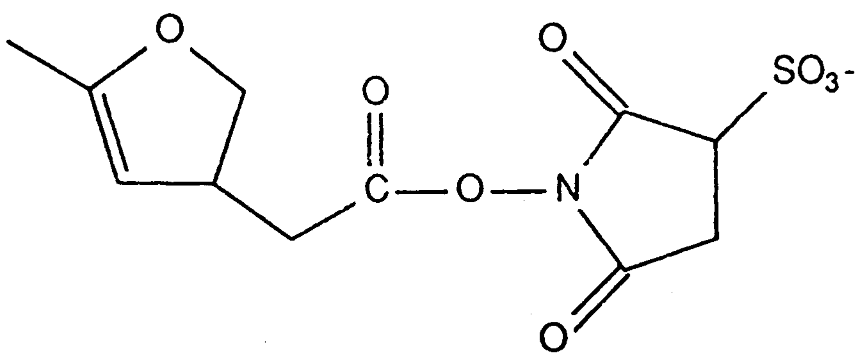

4

2. Coupling of 4 with AH-Sepharose-4B

Aminohexyl (AH)-Sepharose-4B is obtained from Pharmacia and swelling and washing of the gel is carried out according to the procedure in Affinity Chromatography - Principles and Methods, Pharmacia Publication, June 1979, pp. 22-23.

The above sulfosuccinimidate 4 is dissolved in bicarbonate buffer (pH = 8) containing 5-10% acetonitrite at a concentration is excess of the spacer groups. The solution is added to the gel and pH is readjusted if necessary during the period of coupling, which is done overnight. The Sepharose-spacer-4 conjugate is stored at 4 ° C.

B. Isolation of Phenethylmercaptoacetamide With the Derivatized Solid Phase

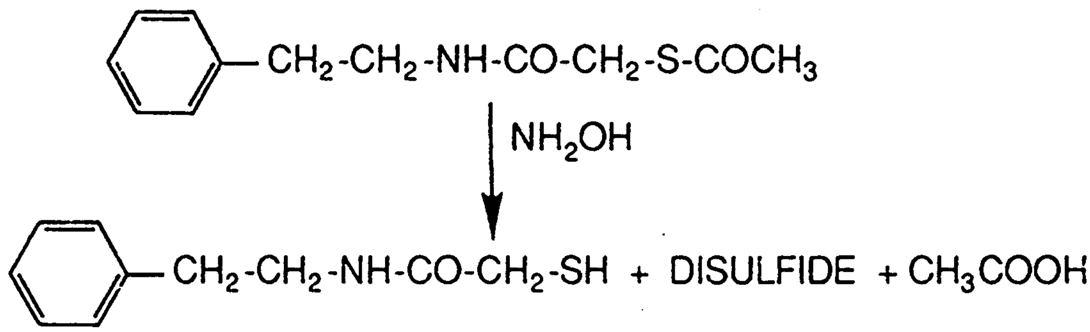

A solution of S-acetylphenethylthioacetamide is treated with excess hydroxylamine in methanol:water (1:1) to yield a mixture of products and unreacted starting material, as depicted above. The solution is passed through a column containing the derivatized Sepharose prepared in part A above. The thiol in the mixture (phenethylmercaptoacetamide) is retained in the column by formation of a covalent adduct, while the other compounds in the mixture (excess hydroxyl amine, the disulfide and unreacted S-acetylphenethylthioacetamide) are eluted.

The desired thiol is freed from the column by passing a pH 4-5 buffer through the column.

The features disclosed in the foregoing description, in the claims and/or in the accompanying drawings may, both separately and in any combination thereof, be material for realising the invention in diverse forms thereof.

## Claims

**1.** A method for producing a cleavable immunoconjugate, comprising the steps of:

reacting an agent, capable of addition to a carbon-carbon double bond, with a compound having the following general formula:

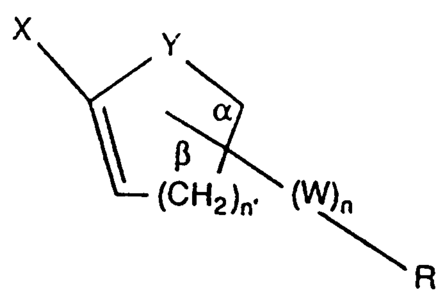

where:
R is a chemically reactive moiety;
W is a methylene, methylenoxy, or methylenecarbonyl group or combinations thereof;
n is 0 to 10;
Y is an O, S or NR', wherein R' is an alkyl group of $C_6$ or less;
n' is 1 to 2; and
X is an H, alkyl group of $C_6$ or less, or alkoxy group of $C_6$ or less;
to form a derivatized agent; and

conjugating said derivatized agent with an antibody or fragment thereof to form said cleavable immunoconjugate.

**2.** The method of claim 1 wherein the agent is selected from the group consisting of drugs, toxins, biological response modifiers, radiodiagnostic compounds, radiotherapeutic compounds, and derivatives thereof.

**3.** The method of claim 2 wherein the toxin is selected from ricin, abrin, diptheria toxin, and <u>Pseudomonas</u>

exotoxin A.

4. The method of claim 1 wherein R is an amino group, sulfhydryl group, hydroxyl group, carbonyl-containing group, or alkyl X', where X' is a leaving group.

5. The method of claim 1, additionally including, after the step of reacting, converting R of the derivatized agent to a group capable of reacting with an antibody or fragment thereof in preparation for conjugation with an antibody or fragment thereof.

6. The method of claim 1, additionally including, after the step of reacting, derivatizing an antibody or fragment thereof in preparation for conjugation with the derivatized agent.

7. The method of claim 1 wherein the antibody or antibody fragment is monoclonal antibody or fragment thereof.

8. The method of claim 1 wherein the antibody fragments are $F(ab')_2$, Fab', Fab, or Fv fragments.

9. The method of claim 1 wherein Y is an O, n' is 1, and the antibody or antibody fragment is a monoclonal antibody or fragment thereof.

10. The method of claim 1 wherein the order of addition of an agent and an antibody or fragment thereof is reversed such that the antibody or fragment thereof is reacted with a compound of claim 1 via R and then the agent is conjugated via the carbon-carbon double bond of the compound.

11. A cleavable immunoconjugate comprising an immunoconjugate having the following general formula:

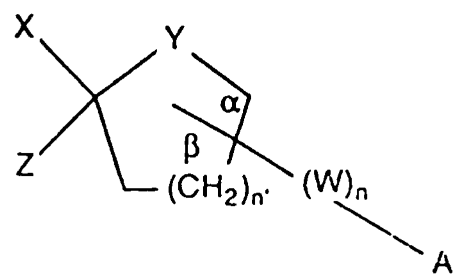

where:
A is an antibody including the linking function, or an antibody fragment including the linking function;
W is a methylene, methylenoxy, or methylenecarbonyl group or combination thereof;
n is 0 to 10;
Y is an O, S or NR', wherein R' is an alkyl group of $C_6$ or less;
n' is 1 to 2;
X is an H, alkyl group of $C_6$ or less, or alkoxy group of $C_6$ or less; and
Z is an agent.

12. The cleavable immunoconjugate of claim 11 wherein the antibody or antibody fragment is a monoclonal antibody or fragment thereof.

13. The cleavable immunoconjugate of claim 11 wherein the antibody fragments are $F(ab')_2$, Fab', Fab, or Fv fragments.

14. The cleavable immunoconjugate of claim 11 wherein the agent is selected from drugs, toxins, biological response modifiers, radiodiagnostic compounds, radiotherapeutic compounds, and derivatives thereof.

15. The cleavable immunoconjugate of claim 14 wherein the toxin is selected from the group consisting of ricin, abrin, diptheria toxin, and Pseudomonas exotoxin A.

**16.** The cleavable immunoconjugate of claim 11 wherein Y is an O, n′ is 1, and the antibody or antibody fragment is a monoclonal antibody or fragment thereof.

**17.** A cleavable immunoconjugate having the following general formula:

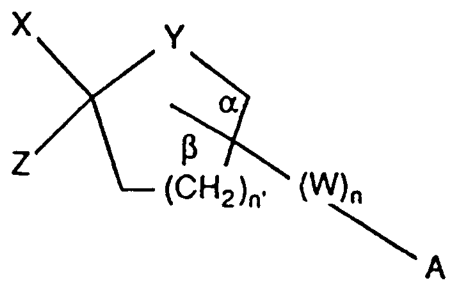

where:
A is an antibody including the linking function, or an antibody fragment including the linking function;
W is a methylene, methylenoxy, or methylenecarbonyl group or combination thereof;
n is 0 to 10;
Y is an O, S or NR', wherein R' is an alkyl group of $C_6$ or less;
n' is 1 to 2;
X is H, alkyl group of $C_6$ or less, or alkoxy group of $C_6$ or less; and
Z is an agent;
for use as an active therapeutic substance.

**18.** A cleavable immunoconjugate having the following general formula:

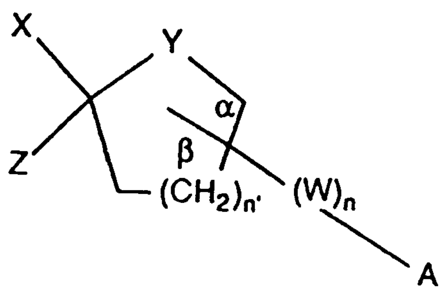

where:
A is an antibody including the linking function, or an antibody fragment including the linking function;
W is a methylene, methylenoxy, or methylenecarbonyl group or combination thereof;
n is 0 to 10;
Y is an O, S or NR', wherein R' is an alkyl group of $C_6$ or less;
n' is 1 to 2;
X is H, alkyl group of $C_6$ or less, or alkoxy group of $C_6$ or less; and
Z is a diagnostically or therapeutically effective agent;
for use within a method for delivering to the cytoplasm of a target cell an agent free of its antibody carrier.

**19.** The immunoconjugate of claim 18 wherein the antibody or antibody fragment is a monoclonal antibody or fragment thereof.

**20.** The immunoconjugate of claim 18 wherein the antibody fragments are $F(ab')_2$, Fab′, Fab, or Fv fragments.

21. The immunoconjugate of claim 18 wherein Y is an O, n′ is 1, and the antibody or antibody fragment is a monoclonal antibody or fragment thereof.

22. The immunoconjugate of claim 18 wherein the agent is selected from drugs, biological response modifiers, radiodiagnostic compounds, radiotherapeutic compounds, toxins, and derivatives thereof.

23. The immunoconjugate of claim 22 wherein the radiodiagnostic or radiotherapeutic compound contains a $^{99m}$Tc, $^{186}$Re, $^{188}$Re, $^{131}$I or $^{123}$I radionuclide.

## Patentansprüche

1. Verfahren zur Herstellung eines spaltbaren Immun-Konjugats, das die Schritte umfaßt:
Zur-Reaktion-Bringen eines Agens, das zur Addition an eine Kohlenstoff-Kohlenstoff-Doppelbindung in der Lage ist, mit einer Verbindung, die die folgende allgemeine Formel besitzt:

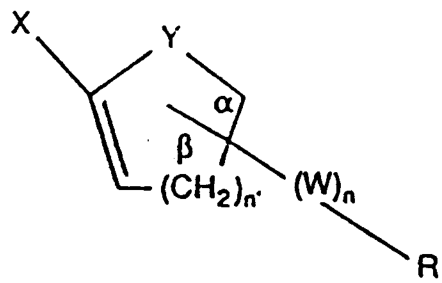

in der:

R eine chemisch reaktive Einheit ist;
W eine Methylen-, Methylenoxy- oder Methylencarbonyl-Gruppe ist oder Kombinationen derselben;
n 0 bis 10 ist;
Y ein O, S oder NR' ist, wobei R' eine Alkylgruppe mit $C_6$ oder weniger ist;
n' 1 bis 2 ist; und
X ein H, eine Alkylgruppe mit $C_6$ oder weniger oder eine Alkoxygruppe mit $C_6$ oder weniger ist;

um ein derivatisiertes Agens zu bilden; und
Konjugieren besagten derivatisierten Agens mit einem Antikörper oder Fragment desselben, um besagtes spaltbares Immun-Konjugat zu bilden.

2. Verfahren nach Anspruch 1, dadurch gekennzeichnet, daß das Agens ausgewählt ist aus der Gruppe, die aus Arzneimitteln, Toxinen, allosterischen Effektoren für biologische Reaktionen, radiodiagnostischen Verbindungen, radiotherapeutischen Verbindungen und Derivaten derselben besteht.

3. Verfahren nach Anspruch 2, dadurch gekennzeichnet, daß das Toxin ausgewählt ist aus Ricin, Abrin, Diptherietoxin und Pseudomonas-Exotoxin A.

4. Verfahren nach Anspruch 1, dadurch gekennzeichnet, daß R eine Aminogruppe, Sulfhydrylgruppe, Hydroxylgruppe, carbonylhaltige Gruppe oder Alkyl-X' ist, wobei X' eine Abgangsgruppe ist.

5. Verfahren nach Anspruch 1, dadurch gekennzeichnet, daß zusätzlich, nach dem Reaktionsschritt, R des derivatisierten Agens in eine Gruppe umgewandelt wird, die in der Lage ist, mit einem Antikörper oder Fragment desselben zu reagieren, in Vorbereitung der Konjugation mit einem Antikörper oder Fragment desselben.

6. Verfahren nach Anspruch 1, dadurch gekennzeichnet, daß zusätzlich, nach dem Reaktionsschritt, ein Antikörper oder Fragment desselben in Vorbereitung der Konjugation mit dem derivatisierten Agens derivatisiert wird.

7. Verfahren nach Anspruch 1, dadurch gekennzeichnet, daß der Antikörper oder das Antikörperfragment ein monoklonaler Antikörper oder Fragment desselben ist.

8. Verfahren nach Anspruch 1, dadurch gekennzeichnet, daß die Antikörperfragmente $F(ab')_2$-, Fab'-, Fab- oder Fv-Fragmente sind.

9. Verfahren nach Anspruch 1, dadurch gekennzeichnet, daß Y ein O ist, n' 1 ist und der Antikörper oder das Antikörperfragment ein monoklonaler Antikörper oder Fragment desselben ist.

10. Verfahren nach Anspruch 1, dadurch gekennzeichnet, daß die Reihenfolge der Zugabe eines Agens und eines Antikörpers oder Fragments desselben umgedreht wird, so daß der Antikörper oder das Fragment desselben mit einer Verbindung nach Anspruch 1 über R zur Reaktion gebracht wird und dann das Agens über die Kohlenstoff-Kohlenstoff-Doppelbindung der Verbindung konjugiert wird.

11. Spaltbares Immun-Konjugat, umfassend ein Immun-Konjugat mit der folgenden allgemeinen Formel:

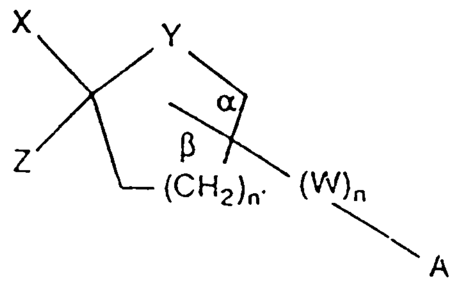

in der:

A ein Antikörper einschließlich der Verknüpfungsfunktion oder ein Antikörperfragment einschließlich der Verknüpfungsfunktion ist;
W eine Methylen-, Methylenoxy- oder Methylencarbonylgruppe oder Kombination derselben ist;
n 0 bis 10 ist;
Y ein O, S oder NR' ist, wobei R' eine Alkylgruppe mit $C_6$ oder weniger ist;
n' 1 bis 2 ist;
X ein H, eine Alkylgruppe mit $C_6$ oder weniger oder eine Alkoxygruppe mit $C_6$ oder weniger ist; und
Z ein Agens ist.

12. Spaltbares Immun-Konjugat nach Anspruch 11, dadurch gekennzeichnet, daß der Antikörper oder das Antikörperfragment ein monoklonaler Antikörper oder Fragment desselben ist.

13. Spaltbares Immun-Konjugat nach Anspruch 11, dadurch gekennzeichnet, daß die Antikörperfragmente $F(ab')_2$-, Fab'-, Fab- oder Fv-Fragmente sind.

14. Spaltbares Immun-Konjugat nach Anspruch 11, dadurch gekennzeichnet, daß das Agens aus Arzneimitteln, Toxinen, allosterischen Effektoren für biologische Reaktionen, radiodiagnostische Verbindungen, radiotherapeutische Verbindungen und Derivaten derselben ausgewählt ist.

15. Spaltbares Immun-Konjugat nach Anspruch 14, dadurch gekennzeichnet, daß das Toxin ausgewählt ist aus der Gruppe, die aus Ricin, Abrin, Diphterietoxin und Pseudomonas-Exotoxin A besteht.

16. Spaltbares Immun-Konjugat nach Anspruch 11, dadurch gekennzeichnet, daß Y ein 0 ist, n' 1 ist und der Antikörper oder das Antikörperfragment ein monoklonaler Antikörper oder Fragment desselben ist.

17. Spaltbares Immun-Konjugat mit der folgenden allgemeinen Formel:

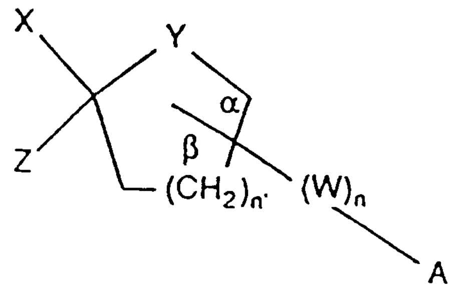

in der:

- A ein Antikörper einschließlich der Verknüpfungsfunktion oder ein Antikörperfragment einschließlich der Verknüpfungsfunktion ist;
- W eine Methylen-, Methylenoxy- oder Methylencarbonylgruppe oder Kombination derselben ist;
- n 0 bis 10 ist;
- Y ein O, S oder NR' ist, wobei R' eine Alkylgruppe mit $C_6$ oder weniger ist;
- n' 1 bis 2 ist;
- X H, eine Alkylgruppe mit $C_6$ oder weniger oder eine Alkoxygruppe mit $C_6$ oder weniger ist; und
- Z ein Agens ist;

zur Verwendung als wirksame therapeutische Substanz.

**18.** Spaltbares Immun-Konjugat mit der folgenden allgemeinen Formel:

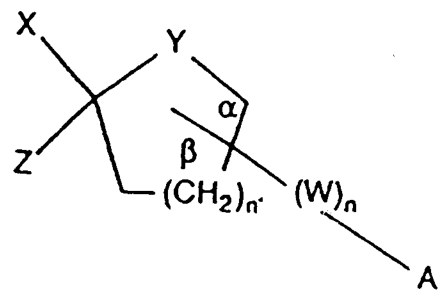

in der:

- A ein Antikörper einschließlich der Verknüpfungsfunktion oder ein Antikörperfragment einschließlich der Verknüpfungsfunktion ist;
- W eine Methylen-, Methylenoxy- oder Methylencarbonylgruppe oder Kombination derselben ist;
- n 0 bis 10 ist;
- Y ein O, S oder NR' ist, wobei R' eine Alkylgruppe mit $C_6$ oder weniger ist;
- n' 1 bis 2 ist;
- X H, eine Alkylgruppe mit $C_6$ oder weniger oder eine Alkoxygruppe mit $C_6$ oder weniger ist; und
- Z ein diagnostisch oder therapeutisch wirksames Agens ist;

zur Verwendung bei einem Verfahren zur Abgabe eines von seinem Antikörper-Träger freien Agens an das Zytoplasma einer Targetzelle.

**19.** Immun-Konjugat nach Anspruch 18, dadurch gekennzeichnet, daß der Antikörper oder das Antikörperfragment ein monoklonaler Antikörper oder Fragment desselben ist.

**20.** Immun-Konjugat nach Anspruch 18, dadurch gekennzeichnet, daß die Antikörperfragmente $F(ab')_2$-, Fab'-, Fab- oder Fv-Fragmente sind.

**21.** Immun-Konjugat nach Anspruch 18, dadurch gekennzeichnet, daß Y ein 0 ist, n 1 ist und der Antikörper oder das Antikörperfragment ein monoklonaler Antikörper oder Fragment desselben ist.

**22.** Immun-Konjugat nach Anspruch 18, dadurch gekennzeichnet, daß das Agens aus Arzneimitteln, allosterischen Effektoren für biologische Reaktionen, radiodiagnostischen Verbindungen, radiotherapeutischen Verbindungen, Toxinen und Derivaten derselben ausgewählt ist.

**23.** Immun-Konjugat nach Anspruch 22, dadurch gekennzeichnet, daß die radiodiagnostische oder radiotherapeutische Verbindung ein $^{99m}$Tc-, $^{186}$Re-, $^{188}$Re-, $^{131}$I- oder $^{123}$I-Radionuklid enthält.

**Revendications**

**1.** Procédé de production d'un immunoconjugué clivable, comprenant les étapes consistant :

à faire réagir un agent, capable de présenter une réaction d'addition à une double liaison carbone-carbone, avec un composé répondant à la formule générale suivante :

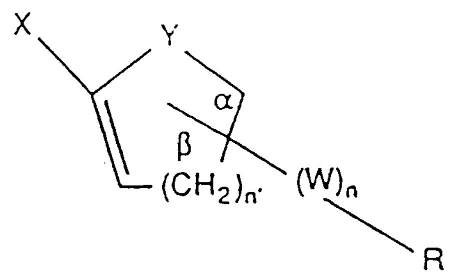

dans laquelle

R représente un groupement chimiquement réactif ;

W représente un groupe méthylène, méthylène-oxy ou méthylène-carbonyle ou une de leurs associations ;

n a une valeur de 0 à 6 ;

Y représente O, S ou un groupe NR', dans lequel R' représente un groupe alkyle ayant un nombre d'atomes de carbone égal ou inférieur à 10 ;

n' est égal à 1 ou 2 ; et

X représente H, un groupe alkyle ayant un nombre d'atomes de carbone égal ou inférieur à 6 ou un groupe alkoxy ayant un nombre d'atomes de carbone égal ou inférieur à 6 ; pour former un dérivé de cet agent ; et

à conjuguer ledit dérivé de cet agent avec un anticorps ou un de ses fragments pour former ledit immunoconjugué clivable.

**2.** Procédé suivant la revendication 1, dans lequel l'agent est choisi dans le groupe comprenant des médicaments, des toxines, des agents de modification de réponse biologique, des composés de radiodiagnostic, des composés radiothérapeutiques et leurs dérivés.

**3.** Procédé suivant la revendication 2, dans lequel la toxine est choisie entre la ricine, l'abrine, la toxine diphtérique et l'exotoxine A de Pseudomonas.

**4.** Procédé suivant la revendication 1, dans lequel R représente un groupe amino, un groupe sulfhydryle, un groupe hydroxyle, un groupe contenant la fonction carbonyle ou un groupe alkyle-X', dans lequel X' représente un groupe partant.

**5.** Procédé suivant la revendication 1, consistant en outre, après l'étape de réaction, à transformer le groupe R du dérivé de l'agent en un groupe capable de réagir avec un anticorps ou un de ses fragments dans une préparation pour la conjugaison avec un anticorps ou un de ses fragments.

**6.** Procédé suivant la revendication 1, consistant en outre, après l'étape de réaction, à transformer en un dérivé un anticorps ou un de ses fragments dans une préparation pour la conjugaison avec le dérivé de l'agent.

**7.** Procédé suivant la revendication 1, dans lequel l'anticorps ou le fragment d'anticorps est un anticorps

monoclonal ou un de ses fragments.

**8.** Procédé suivant la revendication 1, dans lequel les fragments d'anticorps sont des fragments $F(ab')_2$, Fab', Fab ou Fv.

**9.** Procédé suivant la revendication 1, dans lequel Y représente O, n' est égal à 1 et l'anticorps ou le fragment d'anticorps est un anticorps monoclonal ou un de ses fragments.

**10.** Procédé suivant la revendication 1, dans lequel l'ordre d'addition d'un agent et d'un anticorps ou d'un de ses fragments est inversé, de sorte que l'anticorps ou un de ses fragments soit amené à réagir avec un composé suivant la revendication 1 par l'intermédiaire du groupe R et que l'agent soit ensuite conjugué par l'intermédiaire de la double liaison carbone-carbone du composé.

**11.** Immunoconjugué clivable, comprenant un immunoconjugué répondant à la formule générale suivante :

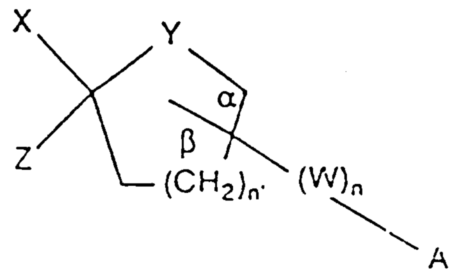

dans laquelle

A représente un anticorps comprenant la fonction de jonction, ou bien un fragment d'anticorps comprenant la fonction de jonction ;

W représente un groupe méthylène, méthylène-oxy ou méthylène-carbonyle ou une de leurs associations ;

n a une valeur de 0 à 10 ;

Y représente O, S ou un groupe NR', dans lequel R' représente un groupe alkyle ayant un nombre d'atomes de carbone égal ou inférieur à 6 ;

n' est égal à 1 ou 2 ; et

X représente H, un groupe alkyle ayant un nombre d'atomes de carbone égal ou inférieur à 6 ou un groupe alkoxy ayant un nombre d'atomes de carbone égal ou inférieur à 6 ; et

Z représente un agent.

**12.** Immunoconjugué clivable suivant la revendication 11, dans lequel l'anticorps ou le fragment d'anticorps est un anticorps monoclonal ou un de ses fragments.

**13.** Immunoconjugué clivable suivant la revendication 11, dans lequel les fragments d'anticorps sont des fragments $F(ab')_2$, Fab', Fab ou Fv.

**14.** Immunoconjugué clivable suivant la revendication 11, dans lequel l'agent est choisi entre des médicaments, des toxines, des agents de modification de réponse biologique, des composés de radiodiagnostic, des composés radiothérapeutiques et leurs dérivés.

**15.** Immunoconjugué clivable suivant la revendication 14, dans lequel la toxine est choisie dans le groupe comprenant la ricine, l'abrine, la toxine diphtérique et l'exotoxine A de Pseudomonas.

**16.** Immunoconjugué clivable suivant la revendication 11, dans lequel Y représente O, n' est égal à 1 et l'anticorps ou le fragment d'anticorps est un anticorps monoclonal ou un de ses fragments.

**17.** Immunoconjugué clivable répondant à la formule générale suivante :

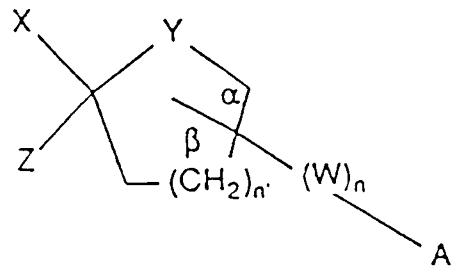

dans laquelle

A représente un anticorps comprenant la fonction de jonction, ou bien un fragment d'anticorps comprenant la fonction de jonction ;

W représente un groupe méthylène, méthylène-oxy ou méthylène-carbonyle ou une de leurs associations ;

n a une valeur de 0 à 10 ;

Y représente O, S ou un groupe NR', dans lequel R' représente un groupe alkyle ayant un nombre d'atomes de carbone égal ou inférieur à 6 ;

n' est égal à 1 ou 2 ; et

X représente H, un groupe alkyle ayant un nombre d'atomes de carbone égal ou inférieur à 6 ou un groupe alkoxy ayant un nombre d'atomes de carbone égal ou inférieur à 6 ; et

Z représente un agent ;

destiné à être utilisé comme substance thérapeutique active.

**18.** Immunoconjugué clivable répondant à la formule générale suivante :

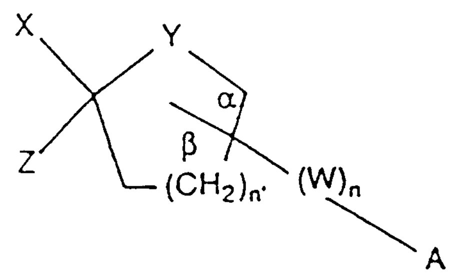

dans laquelle

A représente un anticorps comprenant la fonction de jonction, ou bien un fragment d'anticorps comprenant la fonction de jonction ;

W représente un groupe méthylène, méthylène-oxy ou méthylène-carbonyle ou une de leurs associations ;

n a une valeur de 0 à 10 ;

Y représente O, S ou un groupe NR', dans lequel R' représente un groupe alkyle ayant un nombre d'atomes de carbone égal ou inférieur à 6 ;

n' est égal à 1 ou 2 ; et

X représente H, un groupe alkyle ayant un nombre d'atomes de carbone égal ou inférieur à 6 ou un groupe alkoxy ayant un nombre d'atomes de carbone égal ou inférieur à 6 ; et

Z représente un agent efficace à des fins de diagnostic ou des fins thérapeutiques ; destiné à être utilisé dans un procédé pour fournir au cytoplasme d'une cellule cible un agent dépourvu de son support d'anticorps.

**19.** Immunoconjugué suivant la revendication 18, dans lequel l'anticorps ou le fragment d'anticorps est un anticorps monoclonal ou de ses fragments.

**20.** Immunoconjugué suivant la revendication 18, dans lequel les fragments d'anticorps sont des fragments $F(ab')_2$, Fab', Fab ou Fv.

**21.** Immunoconjugué suivant la revendication 18, dans lequel Y représente O, n est égal à 1 et l'anticorps ou le fragment d'anticorps est un anticorps monoclonal ou l'un de ses fragments.

**22.** Immunoconjugué suivant la revendication 18, dans lequel l'agent est choisi entre des médicaments, des agents de modification de réponse biologique, des composés de radiodiagnostic, des composés radiothérapeutiques, des toxines et leurs dérivés.

**23.** Immunoconjugué suivant la revendication 22, dans lequel le composé de radiodiagnostic ou le composé radiothérapeutique comprend un radionucléide $^{99m}Tc$, $^{186}Re$, $^{188}Re$, $^{131}I$ ou $^{123}I$.